(19) Europäisches Patentamt
European Patent Office
Office européen des brevets

(11) Veröffentlichungsnummer: **0 185 259**
**A2**

(12) # EUROPÄISCHE PATENTANMELDUNG

(21) Anmeldenummer: **85115459.1**

(22) Anmeldetag: **05.12.85**

(51) Int. Cl.⁴: **C 07 D 475/08**
**A 61 K 31/495**

(30) Priorität: **12.12.84 DE 3445298**

(43) Veröffentlichungstag der Anmeldung:
**25.06.86 Patentblatt 86/26**

(84) Benannte Vertragsstaaten:
**AT BE CH DE FR GB IT LI LU NL SE**

(71) Anmelder: **Dr. Karl Thomae GmbH**
**Postfach 1755**
**D-7950 Biberach (Riss)(DE)**

(72) Erfinder: **Roch, Josef, Dr. Dipl.-Chem.**
**Stecherweg 19**
**D-7950 Biberach 1(DE)**

(72) Erfinder: **Heckel, Armin, Dr. Dipl.-Chem.**
**Lamparterweg 1**
**D-7950 Biberach 1(DE)**

(72) Erfinder: **Nickl, Josef, Dr. Dipl.-Chem.**
**Silcherstrasse 8**
**D-7950 Biberach 1(DE)**

(72) Erfinder: **Müller, Erich, Dr. Dipl.-Chem.**
**Talfeldstrasse 34**
**D-7950 Biberach 1(DE)**

(72) Erfinder: **Narr, Berthold, Dr. Dipl.-Chem.**
**Obere Au 5**
**D-7950 Biberach 1(DE)**

(72) Erfinder: **Zimmermann, Rainer, Dr. Dipl.-Biochem.**
**laurenbühlstrasse 17**
**D-7951 Mittelbiberach(DE)**

(72) Erfinder: **Weisenberger, Hans, Dr. Dipl.-Chem.**
**Haydnweg 5**
**D-7950 Biberach 1(DE)**

(54) **Neue Pteridine, Verfahren zu ihrer Herstellung und deren Verwendung als Zwischenprodukte oder als Arzneimittel.**

(57) Die Erfindung betrifft neue Pteridine der allgemeinen Formel

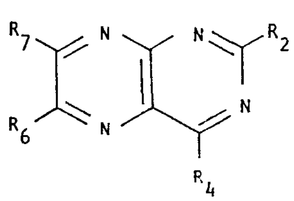

,

in der
$R_2$ die Piperazino- oder N-Formyl-piperazinogruppe,
$R_4$ eine Dialkylamino-, Phenylalkylamino-, N-Alkyl-phenylalkylamino-, Pyrrolidino-, Piperidino-, Morpholino-, Thiomorpholino-, 1-Oxidothiomorpholino-, Thiazolidino- oder 1-Oxidothiazolidinogruppe,
$R_6$ ein Wasserstoffatom, eine Alkyl- oder Phenylgruppe und
$R_7$ eine Alkylaminogruppe, Dialkylamino-, Phenylalkylamino-, N-Alkyl-phenylalkylamino-, Piperidino-, Morpholino-, Thiomorpholino-, 1-Oxidothiomorpholino- oder Piperazinogruppe, wobei in den Resten $R_4$, $R_6$ und $R_7$ jeder Alkylteil jeweils 1 bis 3 Kohlenstoffatome enthalten kann und gleichzeitig eine oder beide Alkylgruppen mit 2 oder 3 Kohlenstoffatomen der Reste $R_4$ und $R_7$ in 2- oder 3-Stellung jeweils durch eine Hydroxygruppe substituiert sein können, bedeuten, und deren Säureadditionssalze.

Diese stellen, wenn $R_2$ die N-Formyl-piperazinogruppe darstellt, wertvolle Zwischenprodukte zur Herstellung der Pteridine der allgemeinen Formel I dar, in der $R_2$ die Piperazinogruppe darstellt, welche wertvolle pharmakologische Eigenschaften aufweisen, insbesondere antithrombotische, metastasenhemmende und eine Hemmwirkung auf das Tumorwachstum.

Die neuen Verbindungen lassen sich nach an für sich bekannten Verfahren herstellen.

EP 0 185 259 A2

Croydon Printing Company Ltd

DR. KARL THOMAE GMBH
D-7950 Biberach 1

Case 5/919
Dr.Fl./Kp.



# Neue Pteridine, Verfahren zu ihrer Herstellung und deren Verwendung als Zwischenprodukte oder als Arzneimittel

In der US-A-2.940.972 werden bereits substituierte Pteridine beschrieben, welche wertvolle pharmakologische Eigenschaften aufweisen, nämlich coronarerweiternde, sedative, antipyretische und analgetische Wirkungen.

Es wurde nun gefunden, daß die neuen Pteridine der allgemeinen Formel

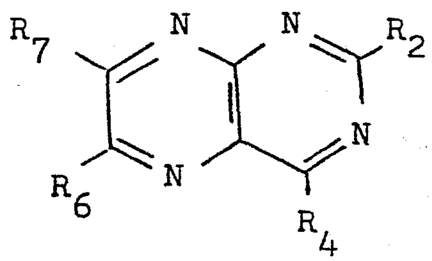

,(I)

und deren Säureadditionssalze wertvolle Eigenschaften aufweisen. Diese stellen, wenn $R_2$ die N-Formyl-piperazinogruppe darstellt, wertvolle Zwischenprodukte zur Herstellung der Pteridine der allgemeinen Formel I, in der $R_2$ die Piperazinogruppe darstellt, und von deren Säureadditionssalze, insbesondere von deren physiologisch verträglichen Säureadditionssalzen mit anorganischen oder organischen Säuren dar, welche wertvolle pharmakologische Eigenschaften aufweisen, insbesondere antithrombotische, metastasenhemmende und eine Hemmwirkung auf das Tumorwachstum.

In der obigen allgemeinen Formel I bedeutet

$R_2$ die Piperazino- oder N-Formyl-piperazinogruppe,

$R_4$ eine Dialkylamino-, Phenylalkylamino-, N-Alkyl-phenylalkylamino-, Pyrrolidino-, Piperidino-, Morpholino-, Thiomorpholino-, 1-Oxidothiomorpholino-, Thiazolidino- oder 1-Oxidothiazolidinogruppe,

$R_6$ ein Wasserstoffatom, eine Alkyl- oder Phenylgruppe und

$R_7$ eine Alkylamino-, Dialkylamino-, Phenylalkylamino-, N-Alkyl-phenylalkylamino-, Piperidino-, Morpholino-, Thiomorpholino-, 1-Oxidothiomorpholino- oder Piperazinogruppe, wobei in den obengenannten Resten $R_4$, $R_6$ und $R_7$ jeder Alkylteil 1 bis 3 Kohlenstoffatome enthalten kann und gleichzeitig eine oder beide Alkylgruppen mit 2 oder 3 Kohlenstoffatomen der Reste $R_4$ und $R_7$ in 2- oder 3-Stellung jeweils durch eine Hydroxygruppe substituiert sein können.

Gegenstand der vorliegenden Erfindung sind somit die als Zwischenprodukte geeigneten neuen 2-(N-Formyl-piperazino)-pteridine der obigen allgemeinen Formel I und deren Säureadditionssalze und die neuen 2-Piperazino-pteridine der obigen allgemeinen Formel I, deren Säureadditionssalze, insbesondere deren physiologisch verträgliche Säureadditionssalze mit anorganischen oder organischen Säuren, Verfahren zu ihrer Herstellung und diese Verbindungen enthaltende Arzneimittel.

Für die bei der Definition der Reste $R_2$, $R_4$, $R_6$ und $R_7$ eingangs erwähnten Bedeutungen kommt beispielsweise

für $R_2$ die der Piperazino- oder N-Formyl-piperazinogruppe,

für $R_4$ die der Dimethylamino-, Diethylamino-, Di-n-propylamino-, Diisopropylamino-, N-Methyl-ethylamino-, N-Ethyl-n-propylamino-, Benzylamino-, N-Methyl-benzylamino-, N-Ethylbenzylamino-, N-n-Propyl-benzylamino-, 1-Phenylethylamino-, 2-Phenylethylamino-, 3-Phenylpropylamino-, N-Methyl-1-phenylethylamino-, N-Ethyl-2-phenylethylamino-, N-Ethyl-3-phenylpropylamino-, Bis(2-hydroxyethyl)-amino-, Bis(2-hydroxy-n-propyl)-amino-, Bis(3-hydroxy-n-propyl)-amino-, N-(2-hydroxyethyl)-2-hydroxy-n-propylamino-, N-Methyl-2-hydroxyethylamino-, N-Ethyl-2-hydroxyethylamino-, N-Methyl-2-hydroxy-n-propylamino-, N-Isopropyl-2-hydroxy-ethylamino-, N-(2-Hydroxyethyl)-benzylamino-, Pyrrolidino-, Piperidino-, Morpholino-, Thiomorpholino-, 1-Oxidothiomorpholino-, Thiazolidino- oder 1-Oxidothiazolidinogruppe,

für $R_6$ die des Wasserstoffatoms, der Methyl-, Ethyl-, n-Propyl-, Isopropyl- oder Phenylgruppe und

für $R_7$ die der Methylamino-, Ethylamino-, n-Propylamino-, Isopropylamino, Dimethylamino-, Diethylamino-, Di-n-propylamino-, Diisopropylamino-, N-Methyl-ethylamino-, N-Ethyl-n-propylamino-, Benzylamino-, N-Methyl-benzylamino-, N-Ethylbenzylamino-, N-n-Propyl-benzylamino-, 1-Phenylethylamino-, 2-Phenylethylamino-, 3-Phenylpropylamino-, N-Methyl-1-phenylethylamino-, N-Ethyl-2-phenylethylamino-, N-Ethyl-3-phenylpropylamino-, 2-Hydroxyethylamino-, 2-Hydroxy-n-propylamino-, 3-Hydroxy-n-propylamino-, 2-Hydroxyisopropylamino-, Bis(2-hydroxyethyl)-amino-, Bis(2-hydroxy-n-propyl)-amino-, Bis(3-hydroxy-n-propyl)-amino-, N-(2-hydroxyethyl)-2-hydroxy-n-propylamino-, N-Methyl-2-hydroxyethylamino-, N-Ethyl-2-hydroxyethylamino-, N-Methyl-2-hydroxy-n-propylamino-, N-Isopropyl-2-hydroxy-ethylamino-, N-(2-Hydroxyethyl)-benzylamino-, Piperidino-, Morpholino-, Thiomorpholino-, 1-Oxidothiomorpholino-, Thiazolidino-, 1-Oxidothiazolidino- oder Piperazinogruppe in Betracht.

Bevorzugte Verbindungen der obigen allgemeinen Formel I sind diejenigen, in denen

$R_2$ die Piperazinogruppe,

$R_4$ die Dimethylamino-, N-Methyl-2-hydroxyethylamino-, Bis(2-hydroxyethyl)-amino-, Benzylamino-, N-Methyl-benzylamino-, Pyrrolidino-, Piperidino-, Morpholino-, Thiomorpholino-, 1-Oxidothiomorpholino-, Thiazolidino- oder 1-Oxidothiazolidinogruppe,

$R_6$ ein Wasserstoffatom, eine Methyl- oder Phenylgruppe und

$R_7$ eine Dimethylamino-, N-Methyl-2-hydroxyethylamino-, Benzylamino-, N-Methyl-benzylamino-, Piperidino-, Morpholino-, Thiomorpholino-, 1-Oxidothiomorpholino- oder Piperazinogruppe bedeuten, und deren Säureadditionssalze, insbesondere deren physiologisch verträgliche Säureadditionssalze.

Besonders bevorzugte Verbindungen der obigen allgemeinen Formel I sind jedoch diejenigen, in den $R_2$ und $R_6$ wie vorstehend erwähnt definiert sind,

$R_4$ eine Pyrrolidino-, Piperidino-, Morpholino-, Thiomorpholino-, 1-Oxidothiomorpholino- oder N-Methyl-2-hydroxyethylaminogruppe und

$R_7$ eine Dimethylamino-, Benzylamino-, N-Methyl-benzylamino-, Morpholino-, Thiomorpholino- oder 1-Oxidothiomorpholinogruppe darstellen.

Erfindungsgemäß erhält man die neuen Verbindungen nach folgenden Verfahren:

a) Umsetzung einer Verbindung der allgemeinen Formel

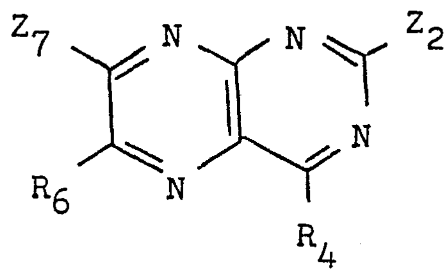

,(II)

in der
$R_4$ und $R_6$ wie eingangs definiert sind,
einer der Reste $Z_2$ oder $Z_7$ eine nukleophil austauschbare Gruppe wie ein Halogenatom, z.B. ein Chlor- oder Bromatom, darstellt und
der andere der Reste $Z_2$ oder $Z_7$ die für $R_2$ oder $R_7$ eingangs erwähnten Bedeutungen besitzt oder, falls eine Verbindung der allgemeinen Formel I hergestellt wird, in der die Reste $R_2$ und $R_7$ die gleiche Bedeutung besitzen, auch eine nukleophil austauschbare Gruppe wie ein Halogenatom, z.B. ein Chlor- oder Bromatom, darstellt, mit einem Amin der allgemeinen Formel

$$H - X \qquad ,(III)$$

in der
X die für $R_2$ oder $R_7$ eingangs erwähnten Bedeutungen besitzt oder eine durch einen hydrolytisch abspaltbaren Schutzrest geschützte Piperazinogruppe darstellt, und erforderlichenfalls anschließende Abspaltung eines verwendeten Schutzrestes.

Die Umsetzung wird zweckmäßigerweise in einem Lösungsmittel wie Tetrahydrofuran, Dioxan, Benzol, Toluol, Dimethylsulfoxid oder Dimethylglycoläther bei Temperaturen zwischen 0 und 150°C, vorzugsweise bei Temperaturen zwischen der Raumtemperatur und der Siedetemperatur des verwendeten Lösungsmittels, oder in der Schmelze durchgeführt. Hierbei kann die Verwendung eines säurebindenden Mittels wie Natriumcarbonat, Triäthylamin oder Pyridin von Vorteil sein.

Die gegebenenfalls erforderliche Abspaltung eines verwendeten Schutzrestes erfolgt entweder hydrolytisch in Gegenwart einer Säure wie Salzsäure oder Schwefelsäure oder einer Base wie Natriumhydroxid oder Kaliumhydroxid vorzugsweise in einem wässrigen Lösungsmittel wie Methanol/Wasser, Äthanol/Wasser oder Dioxan/ Wasser bei Temperaturen bis zur Siedetempertur des verwendeten Lösungsmittels. Die Abspaltung eines verwendeten Schutzrestes kann auch gleichzeitig während der Umsetzung bei Verwendung eines Überschusses des eingesetzten Amins der allgemeinen Formel III erfolgen.

b) Zur Herstellung von Verbindungen der allgemeinen Formel I, in der $R_4$ eine 1-Oxidothiomorpholino- oder 1-Oxidothiazolidinogruppe und/oder $R_7$ eine 1-Oxidothiomorpholinogruppe darstellen:

Oxidation einer Verbindung der allgemeinen Formel

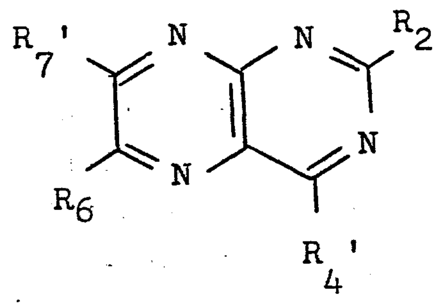

,(IV)

in der
$R_2$ und $R_6$ wie eingangs definiert sind,
$R_4$' und $R_7$' die für $R_4$ bzw. $R_7$ eingangs erwähnten Bedeutung besitzen, wobei jedoch mindestes einer der Reste $R_4$' oder $R_7$' eine Thiomorpholinogruppe oder $R_4$' auch eine Thiazolidinogruppe darstellen muß.

Die Oxidation wird vorzugsweise in einem Lösungsmittel oder Lösungsmittelgemisch, z.B. in Wasser, Wasser/Pyridin, Aceton, Eisessig, Methylenchlorid, Dioxan, verdünnter Schwefelsäure oder Trifluoressigsäure, je nach dem verwendeten Oxidationsmittel zweckmäßigerweise bei Temperaturen zwischen 0 und 150°C durchgeführt.

Die Oxidation wird vorzugsweise mit einem Äquivalent des verwendeten Oxidationsmittels durchgeführt, z.B. mit Wasserstoffperoxid in Eisessig, Trifluoressigsäure oder Ameisensäure bei 0 bis 20°C oder in Aceton bei 0 bis 60°C, mit einer Persäure wie Perameisensäure in Eisessig oder Trifluoressigsäure bei 0 bis 50°C oder mit m-Chlorperbenzoesäure in Methylenchlorid oder Chloroform bei 0 bis 60°C, mit Natriummetaperjodat in Dioxan oder Äthanol bei 80 bis 100°C, mit Brom in Eisessig oder wässriger Essigsäure, mit N-Bromsuccinimid in Äthanol, mit Jodbenzodichlorid in wässrigem Pyridin bei 0 bis 50°C, mit Salpetersäure in Eisessig bei 0 bis 20°C und mit Chromsäure in Eisessig oder in Aceton bei 0 bis 20°C.

Die erfindungsgemäß erhaltenen Verbindungen lassen sich in ihre Säureadditionssalze, insbesondere in ihre physiologisch verträglichen Salze mit anorganischen oder organischen Säuren überführen. Als Säuren kommen beispielsweise Salzsäure, Bromwasserstoffsäure, Schwefelsäure, Phosphorsäure, Milchsäure, Zitronensäure, Weinsäure, Bernsteinsäure, Maleinsäure oder Fumarsäure in Betracht.

Die als Ausgangsstoff verwendeten Verbindungen der allgemeinen Formeln II bis IV sind zum größten Teil bekannt bzw. man erhält diese nach dem in der US-A-2.940.972 beschriebenen Verfahren (siehe Beispiele A bis D).

Wie bereits eingangs erwähnt, weisen die neuen Verbindungen der allgemeinen Formel I, in der $R_2$ die Piperazinogruppe darstellt, und deren Säureadditionssalze, insbesondere deren physiologisch verträgliche Säureadditionssalze mit anorganischen oder organischen Säuren, wertvolle pharmakologische Eigenschaften auf, insbesondere jedoch antithrombotische und

metastasenhemmende Wirkungen und eine Hemmwirkung auf die Phosphodiesterase und auf das Tumorwachstum.

Beispielsweise wurden die Verbindungen

A = 4,7-Dimorpholino-6-phenyl-2-piperazino-pteridin,

B = 4-Morpholino-7-(1-oxidothiomorpholino)-2-piperazino-6-phenyl-pteridin,

C = 7-Benzylamino-6-phenyl-2-piperazino-4-thiomorpholino-pteridin,

D = 7-Dimethylamino-6-phenyl-2-piperazino-4-pyrrolidino-pteridin und

E = 7-Benzylamino-4-(N-methyl-2'-hydroxyethylamino)-2-piperazino-pteridin

auf ihre Hemmwirkung auf die Phosphodiesterase (PDE) von Tumorzellen und von Humanthrombozyten in vitro in Anlehnung an die von Pöch et al. beschriebenen Methode wie folgt untersucht (siehe Naunyn-Schmiedebergs Arch. Pharmak. 268, 272-291 (1971)):

a) Enzymgewinnung:

Die Phosphodiesterase wurde aus B16 Melanomgewebe von Mäusen durch Zentrifugation des Gewebehomogenates bei 5.000 x g (15 min. 4°C) gewonnen. Die Homogenisation der Gewebe erfolgte durch wiederholtes Frieren/Auftauen und Homogenisation nach Potter-Elvehjem bzw. durch Ultraschall. Der die PDE enthaltende Homogenat-Überstand wurde portioniert und bei -25°C tiefgefroren.

Die Gewinnung der Phosphordiesterase aus Humanthrombozyten erfolgte in gleicher Weise.

b) Bestimmung der PDE-Hemmung (PDE-assay):

Die Bestimmung der PDE-Hemmung durch die Prüfsubstanzen erfolgte mit 1 µmol/l $^{3}$H-cAMP als Substrat. Die PDE-Hemmung wurde durch Messung des Abbaus des eingesetzten Substrats $^{3}$H-cAMP zu $^{3}$H-AMP im Vergleich zur Kontrolle ohne Prüfsubstanz erfaßt.
Das gebildete $^{3}$H-AMP wurde durch eine Zinksulfat-Bariumhydroxid-Fällung vom verbliebenen $^{3}$H-cAMP abgetrennt.

Die Berechnung der $IC_{50}$ als die Konzentration, die die PDE-Aktivität um 50 % hemmte, erfolgte mittels linearer Regressionsanalyse.

| Substanz | PDE-Hemmung ($IC_{50}$ in µmol/l) Thrombozyten | B16-Tumorzellen |
|---|---|---|
| A | 0,54 | 4,3 |
| B | 2,2 | 3,1 |
| C | 0,5 | 0,096 |
| D | 3,6 | 0,78 |
| E | 3,7 | 8,3 |

Akute Toxizität:

Die orientierende akute Toxizität der zu untersuchenden Substanzen wurde orientierend an Gruppen von je 10 Mäusen nach oraler Gabe einer Einzeldosis bestimmt (Beobachtungszeit: 14 Tage):

| Substanz | Orientierende aktute Toxizität |
|---|---|
| A | > 250 mg (0 von 5 Tieren gestorben) |
| C | > 250 mg (0 von 5 Tieren gestorben) |
| D | > 250 mg (0 von 5 Tieren gestorben) |
| E | > 250 mg (0 von 5 Tieren gestorben) |

Die erfindungsgemäß hergestellten neuen Verbindungen der allgemeinen Formel I, in der $R_2$ die Piperazinogruppe darstellt, und deren physiologisch verträgliche Säureadditionssalze eignen sich aufgrund ihrer oben erwähnten pharmakologischen Eigenschaften zur Prophylaxe thrombo-embolischer Erkrankungen wie Coronarinfarkt, Cerebralinfarkt, sogn. transient ischaemic attacks, Amaurosis fugax, zur Prophylaxe der Arteriosklerose, zur Metastasenprophylaxe und zur Hemmung des Tumorwachstums.

Die zur Erzielung einer entsprechenden Wirkung erforderlichen Dosierung beträgt zweckmäßigerweise 2- bis 4-mal täglich 0,1 bis 4 mg/kg Körpergewicht, vorzugsweise 0,2 bis 3 mg/kg Körpergewicht. Hierzu lassen sich die erfindungsgemäß hergestellten Verbindungen der allgemeinen Formel I, in der $R_2$ die Piperazinogruppe darstellt, sowie ihre physiologisch verträglichen Säureadditionssalze mit anorganischen oder organischen Säuren, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/- Äthanol, Wasser/-Glycerin, Wasser/Sorbit, nichtionische Tenside wie z.B. Polyoxyäthylen-Fettsäureester, Wasser-Polyäthylenglykol, Propylenglykol, Cetylstearylalkohohl, Carboxymethylcellulose oder fetthaltige Substanzen wie Hartfett oder deren geeignete Gemische, in übliche galenische Zubereitungen wie Tabletten, Dragées, Kapseln, Pulver, Suspensionen, Tropfen, Ampullen, Säfte oder Zäpfchen einarbeiten.

Die nachfolgenden Beispiele sollen die Erfindung näher erläutern:

## Beispiel A

### 2,7-Dichlor-4-morpholino-pteridin

7,03 g (0,03 Mol) 2,4,7-Trichlor-pteridin werden in 100 ml Chloroform gelöst und bei 5°C mit einer Lösung von 3,0 g (0,03 Mol) Kaliumhydrogencarbonat in 50 ml Wasser versetzt. Anschließend tropft man 2,62 g (0,03 Mol) Morpholin in 50 ml Chloroform zu und rührt 45 Minuten bei Raumtemperatur. Danach trennt man die organische Phase ab, trocknet sie über Natriumsulfat und engt im Vakuum ein. Der Rückstand wird aus Ethanol umkristallisiert.
Ausbeute: 7,4 g (86 % der Theorie),
Schmelzpunkt: 187-188°C

Analog Beispiel A werden folgende Verbindungen erhalten:

2,7-Dichlor-4-piperidino-pteridin
Ausbeute: 67 % der Theorie,
Schmelzpunkt: 152-154°C (Ethanol)

2,7-Dichlor-4-benzylamino-pteridin
Ausbeute: 75 % der Theorie,
Schmelzpunkt: 150-152°C (Methanol)

2,7-Dichlor-4-(1-oxidothiomorpholino)-pteridin
Ausbeute: 65 % der Theorie,
Schmelzpunkt: 230-235°C

2,7-Dichlor-4-(N-methyl-2'-hydroxyethylamino)-pteridin
Ausbeute: 74 % der Theorie,
Schmelzpunkt: 178-180°C

2,7-Dichlor-4-diethanolamino-pteridin
Ausbeute: 71 % der Theorie,
Schmelzpunkt: 173-174°C (Ethanol)

2,7-Dichlor-6-methyl-7-morpholino-pteridin
Ausbeute: 73 % der Theorie,
Schmelzpunkt: 230-232°C (Ethanol)

Beispiel B

2,7-Dichlor-6-phenyl-4-thiomorpholino-pteridin

35,5 g (0,114 Mol) 2,4,7-Trichlor-6-phenyl-pteridin werden in 500 ml Aceton gelöst und mit 11,5 g (0,13 Mol) Natriumhydrogencarbonat in 120 ml Wasser versetzt. Dann wird eine Lösung von 11,8 g (0,114 Mol) Thiomorpholin zugegeben und noch 45 Minuten bei Raumtemperatur gerührt. Diese Lösung wird in 2 l Wasser eingegossen, der erhaltene Niederschlag wird gesammelt und aus Ethylenchlorid umkristallisiert.
Ausbeute: 36 g (84 % der Theorie),
Schmelzpunkt: 225-227°C

Analog Beispiel B werden folgende Verbindungen erhalten:

2,7-Dichlor-4-(1-oxidothiomorpholino)-6-phenyl-pteridin
Ausbeute: 88 % der Theorie,
Schmelzpunkt: 222-224°C

2,7-Dichlor-4-morpholino-6-phenyl-pteridin
Ausbeute: 78 % der Theorie,
Schmelzpunkt: 198-201°C

2,7-Dichlor-6-phenyl-4-piperidino-pteridin
Ausbeute: 69 % der Theorie,
Schmelzpunkt: 168-170°C (Essigester)

2,7-Dichlor-4-dimethylamino-6-phenyl-pteridin
Ausbeute: 77 % der Theorie,
Schmelzpunkt: 236-238°C (Ethylenchlorid)

2,7-Dichlor-4-(N-methyl-2'-hydroxyethylamino)-6-phenyl-pteridin
Ausbeute: 76 % der Theorie,
Schmelzpunkt: 162-164°C (Ethanol)

2,7-Dichlor-4-(N-benzyl-methylamino)-6-phenyl-pteridin
Ausbeute: 59 % der Theorie,
Schmelzpunkt: 141-143°C (Ethanol/Dioxan)

2,7-Dichlor-6-phenyl-4-pyrrolidino-pteridin
Ausbeute: 81 % der Theorie,
Schmelzpunkt: 199-200°C (Essigester)

2,7-Dichlor-6-phenyl-4-thiazolidino-pteridin
Ausbeute: 82 % der Theorie,
Schmelzpunkt: 169-171°C (Essigester)

## Beispiel C

7-Chlor-2-(N-formylpiperazino)-6-phenyl-4-thiomorpholino-pteridin

---

Zu einer Lösung von 7,6 g (0,02 Mol) 2,7-Dichlor-6-phenyl-4-thiomorpholino-pteridin in 100 ml Dioxan gibt man 5,5 g (0,048 Mol) N-Formyl-piperazin in 10 ml Dioxan und läßt bei 40°C 1 Stunde rühren. Danach gibt man die Reaktionsmischung in 600 ml Wasser, saugt den Niederschlag ab und kocht ihn mit Essigester aus.

Ausbeute: 88 % der Theorie,
Schmelzpunkt: 223-226°C

Analog Beispiel C werden folgende Verbindungen erhalten:

7-Chlor-4-(N-formylpiperazino)-4-(1-oxidothiomorpholino)-6-phenyl-pteridin
Ausbeute: 90 % der Theorie,
Schmelzpunkt: 230°C (Zersetzung)

7-Chlor-2-(N-formylpiperazino)-4-morpholino-6-phenyl-pteridin
Ausbeute: 90 % der Theorie,
Schmelzpunkt: 247°C (Zersetzung)

7-Chlor-2-(N-formylpiperazino)-6-phenyl-4-piperidino-pteridin
Ausbeute: 66 % der Theorie,
Schmelzpunkt: 210-213°C (Essigester)

7-Chlor-4-dimethylamino-2-(N-formylpiperazino)-6-phenyl-pteridin
Ausbeute: 89 % der Theorie,
Schmelzpunkt: 232-234°C (Essigester)

7-Chlor-2-(N-formylpiperazino)-4-(N-methyl-2'-hydroxyethylamino)-6-phenyl-pteridin
Ausbeute: 86 % der Theorie,
Schmelzpunkt: sintert ab 170°C

7-Chlor-2-(N-formylpiperazino)-4-(N-methyl-benzylamino)-6-phenyl-pteridin
Ausbeute: 63 % der Theorie,
Schmelzpunkt: 143-145°C (Essigester)

7-Chlor-2-(N-formylpiperazino)-6-phenyl-4-pyrrolidino-pteridin
Ausbeute: 60 % der Theorie,
Schmelzpunkt: 210°C (Zersetzung)

7-Chlor-2-(N-formylpiperazino)-6-phenyl-4-thiazolidino-pteridin
Ausbeute: 82 % der Theorie,
Schmelzpunkt: 180-190°C (Essigester)

Beispiel D

2-Chlor-4,7-dimorpholino-pteridin

7,4 g (0,025 Mol) 2,7-Dichlor-4-morpholino-pteridin werden in 150 ml Methylenchlorid gelöst und mit 4,4 g (0,05 Mol) Morpholin 15 Minuten lang bei 40°C gerührt. Anschließend läßt man abkühlen, versetzt mit 200 ml Wasser, trennt die organische Phase ab, trocknet über Natriumsulfat und engt im Vakuum ein. Der Rückstand wird aus Ethanol umkristallisiert.
Ausbeute: 57 % der Theorie,
Schmelzpunkt: 253-255°C

Analog Beispiel D werden folgende Verbindungen erhalten:

7-Benzylamino-2-chlor-4-piperidino-pteridin
Ausbeute: 94 % der Theorie,
Schmelzpunkt: 220-222°C (Dioxan)

4-Benzylamino-2-chlor-7-(1-oxidothiomorpholino)-pteridin
Ausbeute: 45 % der Theorie,
Schmelzpunkt: 264-265°C (Ethanol)

7-Benzylamino-2-chlor-4-(1-oxidothiomorpholino)-pteridin
Ausbeute: 65 % der Theorie,
Schmelzpunkt: 239-240°C (Dioxan/Ethanol)

2-Chlor-4-(N-methyl-2'-hydroxyethylamino)-7-morpholino-pteridin
Ausbeute: 74 % der Theorie,
Schmelzpunkt: 190-192°C

7-Benzylamino-2-chlor-4-(N-methyl-2'-hydroxyethylamino)-pteridin
Ausbeute: 66 % der Theorie,
Schmelzpunkt: 169-170°C

2-Chlor-4-(N-methyl-2'-hydroxyethylamino)-7-thiomorpholino-pteridin
Ausbeute: 80 % der Theorie,
Schmelzpunkt: 214-215°C

7-Benzylamino-2-chlor-4-diethanolamino-pteridin
Ausbeute: 79 % der Theorie,
Schmelzpunkt: 188-189°C (Dioxan)

2-Chlor-4-diethanolamino-7-morpholino-pteridin
Ausbeute: 74 % der Theorie,
Schmelzpunkt: 197-199°C (Ethanol)

2-Chlor-6-methyl-4,7-dimorpholino-pteridin
Ausbeute: 29 % der Theorie,
Schmelzpunkt: 195-200°C

2-Chlor-6-methyl-4-morpholino-7-thiomorpholino-pteridin
Ausbeute: 27 % der Theorie,
Schmelzpunkt: 150-155°C

0185259

## Beispiel 1

### 4,7-Dimorpholino-2-piperazino-pteridin

4,2 g (0,0125 Mol) 2-Chlor-4,7-dimorpholino-pteridin werden in 200 ml Dimethylsulfoxid gelöst und mit einer Lösung von 10,8 g (0,125 Mol) wasserfreiem Piperazin 1 Stunde bei Raumtemperatur gerührt. Dann wird 1 l Wasser zugegeben und mit 2 x 100 ml Methylenchlorid extrahiert. Die organischen Phasen werden über Natriumsulfat getrocknet und einrotiert. Der Rückstand wird mit Ether gewaschen.

Ausbeute: 85 % der Theorie,

Schmelzpunkt: 257-259°C (Zersetzung)

| | | | | | | |
|---|---|---|---|---|---|---|
| Ber.: | C | 55,94 | H | 6,78 | N | 24,00 |
| Gef.: | | 56,26 | | 6,90 | | 23,60 |

$\lambda$max (Ethanol): 380, 279, 248 nm

## Beispiel 2

### 7-Benzylamino-2-piperazino-4-piperidino-pteridin

5,3 g (0,015 Mol) 7-Benzylamino-2-chlor-4-piperidino-pteridin werden in 250 ml Chloroform gelöst und mit einer Lösung von 10,3 g (0,12 Mol) Piperazin am Rückfluß gekocht. Nach 2 Stunden wird mit 200 ml Wasser geschüttelt und die organische Phase über Natriumsulfat getrocknet und im Vakuum eingeengt. Der Rückstand wird aus Dioxan umkristallisiert.

Ausbeute: 79 % der Theorie,

Schmelzpunkt: 185-188°C

| | | | | | | |
|---|---|---|---|---|---|---|
| Ber.: | C | 65,32 | H | 6,98 | N | 27,70 |
| Gef.: | | 65,21 | | 7,35 | | 27,46 |

$\lambda$max (Ethanol): 370, 280, 242 nm

## Beispiel 3

4-Benzylamino-7-(1-oxidothiomorpholino)-2-piperazino-pteridin

Hergestellt analog Beispiel 2 aus 4-Benzylamino-2-chlor-7-(1-oxidothiomorpholino)-pteridin und Piperazin.
Ausbeute: 85 % der Theorie,
Schmelzpunkt: 190-192°C (Methanol)

| | | | | | | | | |
|---|---|---|---|---|---|---|---|---|
| Ber.: | C | 57,51 | H | 5,98 | N | 25,55 | S | 7,31 |
| Gef.: | | 57,35 | | 6,12 | | 25,29 | | 7,24 |

$\lambda$ max (Ethanol): 375, 275, 247 nm

## Beispiel 4

7-Benzylamino-4-(1-oxidothiomorpholino)-2-piperazino-pteridin

Hergestellt analog Beispiel 2 aus 7-Benzylamino-2-chlor-4-(1-oxidothiomorpholino)-pteridin und Piperazin.
Ausbeute: 90 % der Theorie,
Schmelzpunkt: 259-261°C (Methanol)

| | | | | | | | | |
|---|---|---|---|---|---|---|---|---|
| Ber.: | C | 57,52 | H | 5,48 | N | 25,55 | S | 7,31 |
| Gef.: | | 57,46 | | 5,84 | | 25,60 | | 7,66 |

$\lambda$max (Ethanol): 375, 286, 247 nm

## Beispiel 5

4-(N-Methyl-2'-hydroxyethylamino)-7-morpholino-2-piperazino-pteridin

Hergestellt analog Beispiel 2 aus 2-Chlor-4-(N-methyl-2'-hydroxyethylamino)-7-morpholino-pteridin und Piperazin.

Ausbeute: 78 % der Theorie,
Schmelzpunkt: 172-174°C

| | | | | | | |
|---|---|---|---|---|---|---|
| Ber.: | C | 54,53 | H | 7,00 | N | 29,93 |
| Gef.: | | 54,68 | | 7,40 | | 29,64 |

λ max (Ethanol): 384, 278, 247 nm

Beispiel 6

7-Benzylamino-4-(N-methyl-2'-hydroxyethylamino)-2-piperazino-pteridin

Hergestellt analog Beispiel 2 aus 7-Benzylamino-2-chlor-4-(N-methyl-2'-hydroxyethylamino)-pteridin und Piperazin.
Ausbeute: 70 % der Theorie,
Schmelzpunkt: 220-222°C

| | | | | | | |
|---|---|---|---|---|---|---|
| Ber.: | C | 60,84 | H | 6,64 | N | 28,41 |
| Gef.: | | 61,03 | | 6,45 | | 28,12 |

λ max (Ethanol): 372, 277, 243 nm

Beispiel 7

4-(N-Methyl-2'-hydroxyethylamino)-2-piperazino-7-thiomorpholino-pteridin

Hergestellt analog Beispiel 2 aus 2-Chlor-4-(N-methyl-2'-hydroxyethylamino)-7-thiomorpholino-pteridin und Piperazin.
Ausbeute: 65 % der Theorie,
Schmelzpunkt: 175-177°C

| | | | | | | | | |
|---|---|---|---|---|---|---|---|---|
| Ber.: | C | 52,28 | H | 6,71 | N | 28,70 | S | 8,21 |
| Gef.: | | 52,06 | | 6,44 | | 28,42 | | 8,35 |

λ max (Ethanol): 384, 278, 247 nm

Beispiel 8

7-Benzylamino-4-diethanolamino-2-piperazino-pteridin

Hergestellt analog Beispiel 2 aus 7-Benzylamino-2-chlor-4-diethanolamino-pteridin und Piperazin.
Ausbeute: 76 % der Theorie,
Schmelzpunkt: 203-208°C

| | | | | | | |
|---|---|---|---|---|---|---|
| Ber.: | C | 59,42 | H | 6,65 | N | 26,40 |
| Gef.: | | 59,24 | | 6,76 | | 26,24 |

$\lambda$max (Ethanol): 372, 278, 243 nm

Beispiel 9

4-Diethanolamino-7-morpholino-2-piperazino-pteridin

Hergestellt analog Beispiel 2 aus 2-Chlor-4-diethanolamino-7-morpholino-pteridin und Piperazin.
Ausbeute: 62 % der Theorie,
Schmelzpunkt: 187-195°C

| | | | | | | |
|---|---|---|---|---|---|---|
| Ber.: | C | 53,46 | H | 6,98 | N | 27,70 |
| Gef.: | | 53,11 | | 6,94 | | 27,53 |

$\lambda$max (Ethanol): 382, 278, 247 nm

Beispiel 10

6-Methyl-4,7-dimorpholino-2-piperazino-pteridin

Hergestellt analog Beispiel 2 aus 2-Chlor-6-methyl-4,7-dimorpholino-pteridin und Piperazin.
Ausbeute 66 % der Theorie,
Schmelzpunkt: 180-185°C

Ber.: C 56,98 H 7,05 N 27,98
Gef.: 56,78 6,78 27,70
λ max (Ethanol): 382, 279, 248 nm

Beispiel 11

6-Methyl-4-morpholino-2-piperazino-7-thiomorpholino-pteridin

Hergestellt analog Beispiel 2 aus 2-Chlor-6-methyl-4-morpholino-7-thiomorpholino-pteridin und Piperazin.
Ausbeute: 58 % der Theorie,
Schmelzpunkt: 185-188°C
Ber.: C 54,77 H 6,78 N 26,90 S 7,70
Gef.: 54,99 7,05 26,69 7,93
λ max (Ethanol): 382, 280, 247 nm

Beispiel 12

4-(N-Methyl-2'-hydroxyethylamino)-7-(1-oxidothiomorpholino)-2-piperazino-pteridin

2,92 g 4-(N-Methyl-2'-hydroxyethylamino)-7-thiomorpholino-2-piperazino-pteridin werden in 50 ml Methylenchlorid gelöst und mit einer Lösung von 1,72 g m-Chlorperbenzoesäure 2 Stunden bei Raumtemperatur gerührt. Anschließend wird einrotiert und der Rückstand mit Ether gewaschen. Der Rückstand wird in wenig Wasser gelöst, mit 2N Natronlauge alkalisch gestellt und dann mit Methylenchlorid extrahiert. Die organischen Phasen werden getrocknet und einrotiert.
Ausbeute: 1,1 g (36 % der Theorie),
Schmelzpunkt: 223-226°C

| | | | | | | |
|---|---|---|---|---|---|---|
| Ber.: | C | 50,23 | H | 6,45 | S | 7,88 |
| Gef.: | | 49,93 | | 6,42 | | 8,03 |

$\lambda$ max (Ethanol): 386, 278, 245 nm

Beispiel 13

7-Benzylamino-2-(N-formylpiperazino)-6-phenyl-4-thiomorpholino-pteridin

3,2 g (7 mMol) 7-Chlor-2-(N-formylpiperazino)-6-phenyl-4-thiomorpholino-pteridin werden mit 2,2 g (21 mMol) Benzylamin in 15 ml Dioxan am Rückfluß erhitzt. Nach 3 Stunden wird das Lösungsmittel im Vakuum abgezogen und der ölige Rückstand in 100 ml Wasser gegossen. Das ausgefallene Produkt wird gesammelt, getrocknet und über eine kurze Kieselgelsäule mit Essigsäureethylester/Methanol 15:1 chromatographiert. Anschließend wird die Substanz aus Essigester umkristallisiert.
Ausbeute: 80 % der Theorie,
Schmelzpunkt: 218-221°C

Analog Beispiel 13 werden folgende Verbindungen erhalten:

2-(N-Formylpiperazino)-7-(N-methyl-2'-hydroxyethylamino)-6-phenyl-4-thiomorpholino-pteridin
Ausbeute: 55 % der Theorie,
Schmelzpunkt: sintert ab 130°C (Essigester)

2-(N-Formylpiperazino)-4-morpholino-7-(1-oxidothiomorpholino)-6-phenyl-pteridin
Ausbeute: 50 % der Theorie,
Schmelzpunkt: 250-252°C (Wasser)

2-(N-Formylpiperazino)-4-(N-methyl-2'-hydroxyethylamino)-7-morpholino-6-phenyl-pteridin
Ausbeute: 80 % der Theorie,
Schmelzpunkt: 165-175°C

2-(N-Formylpiperazino)-7-(N-methyl-benzylamino)-4-(N-methyl-2'-hydroxy-ethylamino)-6-phenyl-pteridin
Ausbeute: 80 % der Theorie.
Schmelzpunkt: 135-155°C

2-(N-Formylpiperazino)-4-(N-methyl-2'-hydroxyethylamino)-7-thiomorpholino-6-phenyl-pteridin
Ausbeute: 80 % der Theorie,
Schmelzpunkt: sintert ab 155°C

4,7-Di-(N-methyl-benzylamino)-2-(N-formylpiperazino)-6-phenyl-pteridin
Ausbeute: 50 % der Theorie,
Schmelzpunkt: 50-70°C

7-Dimethylamino-2-(N-formylpiperazino)-4-(N-methyl-benzyl-amino)-6-phenyl-pteridin
Ausbeute: 90 % der Theorie,
Schmelzpunkt: sintert ab 90°C

2-(N-Formylpiperazino)-4-(N-methyl-benzylamino)-7-morpholino-6-phenyl-pteridin
Ausbeute: 80 % der Theorie,
Schmelzpunkt: sintert ab 40°C

## Beispiel 14

7-Benzylamino-6-phenyl-2-piperazino-4-thiomorpholino-pteridin

1,58 g (3 mMol) 7-Benzylamino-2-(N-formylpiperazino)-6-phenyl-4-thiomorpholino-pteridin werden mit 15 ml 10%iger Salzsäure 30 Minuten lang am Rückfluß erhitzt. Nach Abkühlen versetzt man die Lösung mit wässriger Kaliumcarbonatlösung und extrahiert das Produkt mit Chloroform. Die organische Phase wird im Vakuum eingeengt und das Produkt über eine Kieselgelsäure mit Methanol/konz. Ammoniak 80:1 chromatographiert.

Ausbeute: 0,8 g (54 % der Theorie),

Schmelzpunkt: 115-130°C

| | | | | | | |
|---|---|---|---|---|---|---|
| Ber.: | C | 65,03 | H | 6,06 | N | 22,47 |
| Gef.: | | 65,20 | | 6,22 | | 22,07 |

$\lambda$max (Ethanol): 386, 292, 248 nm

## Beispiel 15

7-(N-Methyl-2'-hydroxyethylamino)-6-phenyl-2-piperazino-4-thiomorpholino-pteridin

Hergestellt aus 2-(N-Formylpiperazino)-7-(N-methyl-2'-hydroxyethylamino)-6-phenyl-4-thiomorpholino-pteridin analog Beispiel 14.

Ausbeute: 52 % der Theorie,

Schmelzpunkt: sintert ab 70°C

| | | | | | | |
|---|---|---|---|---|---|---|
| Ber.: | C | 59,20 | H | 6,48 | N | 24,02 |
| Gef.: | | 59,44 | | 6,22 | | 23,85 |

$\lambda$ max (Ethanol): 400, 297, 258 nm

## Beispiel 16

4-Morpholino-7-(1-oxidothiomorpholino)-6-phenyl-2-piperazino-pteridin

Hergestellt aus 2-(N-Formylpiperazino)-4-morpholino-7-(1-oxidothiomorpholino)-6-phenyl-pteridin analog Beispiel 14.
Ausbeute: 43 % der Theorie.
Schmelzpunkt: 196-199°C

| | | | | | | | | |
|---|---|---|---|---|---|---|---|---|
| Ber.: | C | 58,28 | H | 6,11 | N | 22,66 | S | 6,48 |
| Gef.: | | 57,93 | | 6,31 | | 22,72 | | 6,44 |

λmax (Ethanol): 400, 295, 258 nm

## Beispiel 17

4-(N-Methyl-2'-hydroxyethylamino)-7-morpholino-6-phenyl-2-piperazino-pteridin

Hergestellt aus 2-(N-Formylpiperazino)-4-(N-methyl-2'-hydroxyethylamino)-7-morpholino-6-phenyl-pteridin analog Beispiel 14.
Ausbeute: 31 % der Theorie.
Schmelzpunkt: sintert ab 90°C

| | | | | | | |
|---|---|---|---|---|---|---|
| Ber.: | C | 61,30 | H | 6,71 | N | 24,87 |
| Gef.: | | 61,52 | | 6,73 | | 24,75 |

λmax (Ethanol): 398, 296, 258 nm

## Beispiel 18

7-(N-Methyl-benzylamino)-4-(N-methyl-2'-hydroxyethylamino)-6-phenyl-2-piperazino-pteridin

Hergestellt aus 2-(N-Formylpiperazino)-7-(N-methyl-benzylamino)-4-(N- methyl-2'-hydroxyethylamino)-6-phenyl-pteridin analog Beispiel 14.
Ausbeute: 19 % der Theorie,
Schmelzpunkt: 80-110°C

| | | | | | | |
|---|---|---|---|---|---|---|
| Ber.: | C | 66,91 | H | 6,66 | N | 23,13 |
| Gef.: | | 66,80 | | 6,58 | | 22,70 |

$\lambda$max (Ethanol): 395, 294, 258 nm

## Beispiel 19

4-(N-Methyl-2'-hydroxyethylamino)-6-phenyl-2-piperazino-7-thiomorpholino-pteridin

Hergestellt aus 2-(N-Formylpiperazino)-4-(N-methyl-2'-hydroxyethylamino)-6-phenyl-7-thiomorpholino-pteridin analog Beispiel 14.

Ausbeute: 13 % der Theorie,

Schmelzpunkt: 100-140°C

| | | | | | | |
|---|---|---|---|---|---|---|
| Ber.: | C | 59,20 | H | 6,48 | N | 24,02 |
| Gef.: | | 59,66 | | 6,45 | | 23,74 |

$\lambda$max (Ethanol): 398, 296, 258 nm

## Beispiel 20

4,7-Bis-(N-methyl-benzylamino)-6-phenyl-2-piperazino-pteridin

Hergestellt aus 2-(N-Formylpiperazino)-4,7-bis-(N-methyl-benzylamino)-6-phenyl-pteridin analog Beispiel 14.

Ausbeute: 56 % der Theorie,

Schmelzpunkt: Harzartiges Produkt

| | | | | | | |
|---|---|---|---|---|---|---|
| Ber.: | C | 72,47 | H | 6,46 | N | 21,12 |
| Gef.: | | 72,60 | | 6,62 | | 20,90 |

$\lambda$max (Ethanol): 398, 294, 258 nm

Beispiel 21

7-Dimethylamino-4-(N-methyl-benzylamino)-6-phenyl-2-piperazino-pteridin

Hergestellt aus 2-(N-Formylpiperazino)-7-dimethylamino-4-(N-methyl-benzylamino)-6-phenyl-pteridin analog Beispiel 14.
Ausbeute: 69 % der Theorie,
Schmelzpunkt: 129-132°C (Essigester)

| | | | | | | |
|---|---|---|---|---|---|---|
| Ber.: | C | 68,69 | H | 6,65 | N | 24,65 |
| Gef.: | | 68,84 | | 6,81 | | 24,37 |

λ max (Ethanol): 396, 294, 258 nm

Beispiel 22

4-(N-Methyl-benzylamino)-7-morpholino-6-phenyl-2-piperazino-pteridin

Hergestellt aus 2-(N-Formylpiperazino)-4-(N-methyl-benzylamino)-7-morpholino-6-phenyl-pteridin analog Beispiel 14.
Ausbeute: 60 % der Theorie,
Schmelzpunkt: 70-100°C

| | | | | | | |
|---|---|---|---|---|---|---|
| Ber.: | C | 67,71 | H | 6,50 | N | 22,57 |
| Gef.: | | 67,96 | | 6,49 | | 22,55 |

λ max (Ethanol): 396, 294, 258 nm

Beispiel 23

7-Dimethylamino-6-phenyl-2-piperazino-4-pyrrolidino-pteridin

Hergestellt aus 2-(N-Formylpiperazino)-7-dimethylamino-6-phenyl-4-pyrrolidino-pteridin analog Beispiel 14.

Ausbeute: 30 % der Theorie,
Schmelzpunkt: 125-130°C

| | | | | | | |
|---|---|---|---|---|---|---|
| Ber.: | C | 59,20 | H | 6,48 | N | 24,02 |
| Gef.: | | 59,44 | | 6,71 | | 23,65 |

λmax (Ethanol): 394, 294, 257 nm

Beispiel 24

7-Morpholino-6-phenyl-2-piperazino-4-thiazolidino-pteridin

Hergestellt aus 2-(N-Formylpiperazino)-7-morpholino-6-phenyl-4-thiazolidino-pteridin analog Beispiel 14.
Ausbeute: 56 % der Theorie,
Schmelzpunkt: 120-150°C

| | | | | | | | | |
|---|---|---|---|---|---|---|---|---|
| Ber.: | C | 59,46 | H | 6,07 | N | 24,12 | S | 6,90 |
| Gef.: | | 59,13 | | 6,13 | | 24,36 | | 6,68 |

λmax (Ethanol): 396, 294, 258 nm

Beispiel 25

7-Morpholino-4-(1-oxidothiazolidino)-6-phenyl-2-piperazino-pteridin

0,93 g (2 mMol) 7-Morpholino-6-phenyl-2-piperazino-4-thiazolidino-pteridin werden in 15 ml Dioxan gelöst und mit 0,86 g Natriummetaperiodat in 5 ml Wasser 1,5 Stunden unter Rückfluß erhitzt. Anschließend wird einrotiert, der Rückstand mit Chloroform aufgenommen und über eine Kieselgelsäule mit Ethanol/Ammoniak 25:1 chromatographiert.
Ausbeute: 31 % der Theorie,
Schmelzpunkt: 190-250°C

| | | | | | | |
|---|---|---|---|---|---|---|
| Ber.: | C | 57,49 | H | 5,87 | S | 6,67 |
| Gef.: | | 57,24 | | 6,10 | | 7,15 |

max (Ethanol): 394, 290, 258 nm

Beispiel 26

4-(N-Methyl-2'-hydroxyethylamino)-7-(1-oxidothiomorpholino)-6-phenyl-2-piperazino-pteridin

Hergestellt analog Beispiel 25 aus 4-(N-Methyl-2'-hydroxyethylamino)-7-thiomorpholino-6-phenyl-2-piperazino-pteridin.
Ausbeute: 58 % der Theorie.
Schmelzpunkt: sintert ab 110°C

| | | | | | | |
|---|---|---|---|---|---|---|
| Ber.: | C | 57,25 | H | 6,27 | S | 6,64 |
| Gef.: | | 57,45 | | 6,59 | | 6,96 |

max (Ethanol): 396, 296, 255 nm

Beispiel 27

4,7-Bis-dimethylamino-6-phenyl-2-piperazino-pteridin

Hergestellt aus 4,7-Bis-dimethylamino-2-(N-formyl-piperazino)-6-phenyl-pteridin analog Beispiel 14.
Ausbeute: 85 % der Ausbeute.
Schmelzpunkt: sintert ab 50°C

| | | | | | | |
|---|---|---|---|---|---|---|
| Ber.: | C | 63,46 | H | 6,93 | N | 29,61 |
| Gef.: | | 63,22 | | 7,07 | | 29,82 |

$\lambda$ max (Ethanol): 394, 293, 257 nm

Beispiel 28

6-Phenyl-2-piperazino-4,7-dipiperidino-pteridin

Hergestellt aus 2-(N-Formylpiperazino)-6-phenyl-4,7-dipiperidino-pteridin analog Beispiel 14.
Ausbeute: 43 % der Theorie.
Schmelzpunkt: sintert ab 90°C

| | | | | | | |
|---|---|---|---|---|---|---|
| Ber.: | C | 68,10 | H | 7,47 | N | 24,43 |
| Gef.: | | 68,00 | | 7,47 | | 24,29 |

λmax (Ethanol): 400, 299, 260 nm

Beispiel 29

2,7-Dipiperazino-4-morpholino-6-phenyl-pteridin

25,2 g (0,07 Mol) 2,7-Dichlor-4-morpholino-6-phenyl-pteridin werden in 70 ml Ethanol mit 36 g Piperazin 1 Stunde bei Raumtemperatur gerührt. Anschließend wird das Gemisch in 700 ml Wasser eingerührt, der entstandene Niederschlag abgesaugt, in 4 N Essigsäure gelöst und mit 8 N Natronlauge wieder gefällt.
Ausbeute: 24 g (74 % der Theorie),
Schmelzpunkt: sintert ab 130°C

| | | | | | | |
|---|---|---|---|---|---|---|
| Ber.: | C | 62,44 | H | 6,77 | N | 27,31 |
| Gef.: | | 62,78 | | 6,70 | | 27,31 |

λmax (Ethanol): 400, 298, 260 nm

Beispiel 30

2,7-Dipiperazino-4-thiomorpholino-6-phenyl-pteridin

Hergestellt aus 2,7-Dichlor-4-thiomorpholino-6-phenyl-pteridin analog Beispiel 29.
Ausbeute: 79 % der Theorie,
Schmelzpunkt: sintert ab 80°C

| | | | | | | |
|---|---|---|---|---|---|---|
| Ber.: | C | 60,35 | H | 6,54 | N | 6,71 |
| Gef.: | | 60,20 | | 6,67 | | 6,92 |

λmax (Ethanol): 400, 299, 259 nm

Beispiel 31

7-Morpholino-4-(1-oxidothiomorpholino)-6-phenyl-2-piperazino-pteridin

4,6 g (0,01 Mol) 7-Chlor-2-(N-formylpiperazino)-4-(1-oxidothiomorpholino)-6-phenyl-pteridin werden in 8,7 g (0,1 Mol) Morpholin 1 Stunde bei 100°C erhitzt. Die Lösung wird im Vakuum eingeengt und der Rückstand über eine Kieselgelsäule mit Methanol/Ammoniak 50:1 chromatographiert.
Ausbeute: 1,5 g (30 % der Theorie),
Schmelzpunkt: 151-154°C (Methanol)

| | | | | | | | | |
|---|---|---|---|---|---|---|---|---|
| Ber.: | C | 58,28 | H | 6,11 | N | 22,66 | S | 6,48 |
| Gef.: | | 57,99 | | 6,13 | | 22,22 | | 6,65 |

λmax (Ethanol): 398, 294, 258 nm

## Beispiel 32

7-Benzylamino-4-(1-oxidothiomorpholino)-6-phenyl-2-piperazino-pteridin

Hergestellt aus 7-Chlor-2-(N-formyl-piperazino)-4-(1-oxidothiomorpholino)-6-phenyl-pteridin und Benzylamin analog Beispiel 31.
Ausbeute: 50 % der Theorie,
Schmelzpunkt: 200°C (Zersetzung)

| | | | | | | | | |
|---|---|---|---|---|---|---|---|---|
| Ber.: | C | 63,01 | H | 5,88 | N | 21,77 | S | 6,23 |
| Gef.: | | 62,89 | | 6,07 | | 21,45 | | 6,20 |

## Beispiel 33

7-Benzylamino-4-morpholino-6-phenyl-2-piperazino-pteridin

Hergestellt aus 7-Chlor-2-(N-formyl-piperazino)-4-morpholino-6-phenyl-pteridin und Benzylamin analog Beispiel 31.
Ausbeute: 35 % der Theorie,
Schmelzpunkt: 135-138°C (Essigester)

| | | | | | | |
|---|---|---|---|---|---|---|
| Ber.: | C | 67,20 | H | 6,27 | N | 23,22 |
| Gef.: | | 67,25 | | 6,34 | | 23,42 |

$\lambda$ max (Ethanol): 385, 290, 248 nm

## Beispiel 34

4,7-Dimorpholino-6-phenyl-2-piperazino-pteridin

Hergestellt aus 7-Chlor-2-(N-formyl-piperazino)-4-morpholino-6-phenyl-pteridin und Morpholin analog Beispiel 31.

Ausbeute: 13 % der Theorie,

Schmelzpunkt: 220-223°C

| | | | | | | |
|---|---|---|---|---|---|---|
| Ber.: | C | 62,32 | H | 6,54 | N | 24,23 |
| Gef.: | | 62,21 | | 6,64 | | 24,05 |

$\lambda$ max (Ethanol): 400, 296, 259 nm

Beispiel 35

4,7-Bis-(1-Oxidothiomorpholino)-6-phenyl-2-piperazino-pteridin

Hergestellt aus 7-Chlor-2-(N-formyl-piperazino)-4-(1-oxidothiomorpholino)-6-phenyl-pteridin und 1-Oxidothiomorpholin analog Beispiel 31.

Ausbeute: 25 % der Theorie.

Schmelzpunkt: 250°C (Zersetzung)

| | | | | | | |
|---|---|---|---|---|---|---|
| Ber.: | C | 54,73 | H | 5,74 | N | 24,28 |
| Gef.: | | 54,60 | | 5,71 | | 24,04 |

$\lambda$ max (Ethanol): 400, 296, 259 nm

Beispiel I

Dragées mit 4 mg 7-Benzylamino-6-phenyl-2-piperazino-4-thiomorpholino-pteridin

Zusammensetzung:

1 Drageekern enthält:

| | | |
|---|---|---|
| Wirksubstanz | (1) | 4,0 mg |
| Milchzucker | (2) | 27,0 mg |
| Maisstärke | (3) | 14,5 mg |
| Polyvinylpyrrolidon | (4) | 4,0 mg |
| Magnesiumstearat | (5) | 0,5 mg |
| | | 50,0 mg |

Herstellung:

Die Stoffe 1-3 werden mit einer wäßrigen Lösung von 4 gleichmäßig befeuchtet, durch 1 mm-Maschenweite gesiebt, getrocknet und erneut durch 1 mm-Maschenweite gesiebt. Nach Zumischen von 5 wird die Mischung zu Drageekernen verpreßt.

Drageekerne: 5 mm 0, bikonvex, rund

Dragierung:

Übliche Zuckerdragierung auf 70 mg Endgewicht.

Beispiel II

Tabletten mit 8 mg 7-Benzylamino-6-phenyl-2-piperazino-4-thiomorpholino-pteridin

1 Tablette enthält:

| | |
|---|---|
| Wirksubstanz | 8,0 mg |
| Milchzucker | 23,0 mg |

| | |
|---|---|
| Maisstärke | 14,5 mg |
| Polyvinylpyrrolidon | 4,0 mg |
| Magnesiumstearat | 0,5 mg |
| | 50,0 mg |

Herstellung:

Analog den Dragéekernen.

Tablettenbeschreibung:

Gewicht: 50 mg
Durchmesser: 5 mm, biplan, beidseitige Facette

Beispiel III

Suppositorien zu 25 mg 7-Benzylamino-6-phenyl-2-piperazino-4-thiomorpholino-pteridin

1 Zäpfchen enthält:

| | |
|---|---|
| Wirksubstanz | 0,025 g |
| Hartfett (z.B. Witepsol H 19 | 1,695 g |
| und Witepsol H 45) | 1,700 g |

Herstellung:

Das Hartfett wird geschmolzen. Bei 38°C wird die gemahlene Wirksubstanz in der Schmelze homogen dispergiert. Es wird auf 35°C abgekühlt und in schwach vorgekühlte Suppositorienformen ausgegossen.

Zäpfchengewicht: 1,7 g

Beispiel IV

Suspension mit 8 mg 7-Benzylamino-6-phenyl-2-piperazino-4-thiomorpholino-pteridin pro 5 ml

100 ml Suspension enthalten:

| | | |
|---|---|---|
| Wirksubstanz | | 0,16 g |
| Carboxymethylcellulose | | 0,1 g |
| p-Hydroxybenzoesäuremethylester | | 0,05 g |
| p-Hydroxybenzoesäurepropylester | | 0,01 g |
| Rohrzucker | | 10,0 g |
| Glycerin | | 5,0 g |
| Sorbitlösung 70 % | | 20,0 g |
| Aroma | | 0,3 g |
| Wasser dest. | ad | 100.0 ml |

Herstellungsverfahren:

Dest. Wasser wird auf 70°C erhitzt. Hierin wird unter Rühren p-Hydroxybenzoesäuremethylester und -propylester sowie Glycerin und Carboxymethylcellulose gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.

Beispiel V

Tabletten mit 100 mg 7-Benzylamino-6-phenyl-2-piperazino-4-thiomorpholino-pteridin

Zusammensetzung:
1 Tablette enthält:

| | |
|---|---|
| Wirkstoff | 100,0 mg |
| Milchzucker | 80,0 mg |

| | |
|---|---|
| Maisstärke | 34,0 mg |
| Polyvinylpyrrolidon | 4,0 mg |
| Magnesiumstearat | 2,0 mg |
| | 220,0 mg |

Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.

Tablettengewicht: 220 mg
Durchmesser: 10 mm, biplan mit beidseitiger Facette und einseitiger Teilkerbe.

Beispiel VI

Hartgelatine-Kapseln mit 150 mg 7-Benzylamino-6-phenyl-2-piperazino-4-thiomorpholino-pteridin

1 Kapsel enthält:

| | | |
|---|---|---|
| Wirkstoff | | 150,0 mg |
| Maisstärke getr. | ca. | 180,0 mg |
| Milchzucker pulv. | ca. | 87,0 mg |
| Magnesiumstearat | | 3,0 mg |
| | ca. | 320,0 mg |

Herstellung:

Der Wirkstoff wird mit den Hilfsstoffen vermengt, durch ein Sieb von 0,75 mm-Maschenweite gegeben und in einem geeigneten Gerät homogen gemischt.

Die Endmischung wird in Hartgelatine-Kapseln der Größe 1 abgefüllt.

Kapselfüllung: ca. 320 mg
Kapselhülle: Hartgelatine-Kapsel Größe 1.

## Beispiel VII

Suppositorien mit 150 mg 7-Benzylamino-6-phenyl-2-piperazino-4-thiomorpholino-pteridin

1 Zäpfchen enthält:

| | |
|---|---|
| Wirkstoff | 150,0 mg |
| Polyäthylenglykol 1500 | 550,0 mg |
| Polyäthylenglykol 6000 | 460,0 mg |
| Polyoxyäthylensorbitanmonostearat | 840,0 mg |
| | 2 000,0 mg |

### Herstellung:

Nach dem Aufschmelzen der Suppositorienmasse wird der Wirkstoff darin homogen verteilt und die Schmelze in vorgekühlte Formen gegossen.

## Beispiel VIII

Suspension mit 50 mg 7-Benzylamino-6-phenyl-2-piperazino-4-thiomorpholino-pteridin pro 5 ml

100 ml Suspension enthalten:

| | |
|---|---|
| Wirkstoff | 1,0 g |
| Carboxymethylcellulose-Na-Salz | 0,1 g |
| p-Hydroxybenzoesäuremethylester | 0,05 g |
| p-Hydroxybenzoesäurepropylester | 0,01 g |

| | | | |
|---|---|---|---|
| Rohrzucker | | 10,0 | g |
| Glycerin | | 5,0 | g |
| Sorbitlösung 70%ig | | 20,0 | g |
| Aroma | | 0,3 | g |
| Wasser dest. | ad | 100 | ml |

Herstellung:

Dest. Wasser wird auf 70°C erhitzt. Hierin wird unter Rühren p-Hydroxybenzoesäuremethylester und -propylester wobei Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.

5 ml Suspension enthalten 50 mg Wirkstoff.

## Beispiel IX

Tabletten mit 150 mg 7-Benzylamin-6-phenyl-2-piperazino-4-thiomorpholino-pteridin

Zusammensetzung:
1 Tablette enthält:

| | |
|---|---|
| Wirksubstanz | 150,0 mg |
| Milchzucker pulv. | 89,0 mg |
| Maisstärke | 40,0 mg |
| Kolloide Kieselgelsäure | 10,0 mg |
| Polyvinylpyrrolidon | 10,0 mg |
| Magnesiumstearat | 1,0 mg |
| | 300,0 mg |

Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen.
Das bei 45°C getrocknete Granulat wird nochmals durch dasselbe Sieb gerieben und mit der angegebenen Menge Magnesiumstearat gemischt. Aus der Mischung werden Tabletten gepreßt.

Tablettengewicht: 300 mg
Stempel: 10 mm, flach

Beispiel X

Dragées mit 75 mg 7-Benzylamino-6-phenyl-2-piperazino-4-thiomorpholino-pteridin

1 Dragéekern enthält:

| | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 230,0 mg |

Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepreßt.

Kerngewicht: 230 mg
Stempel: 9 mm, gewölbt

0185259

Die so hergestellten Dragéekerne werden mit einem Film überzogen, der im wesentlichen aus Hydroxypropylmethylcellulose besteht. Die fertigen Filmdragées werden mit Bienenwachs geglänzt.

Dragéegewicht: 245 mg.

Selbstverständlich können alle übrigen Verbindungen der allgemeinen Formel I als Wirkstoffe in den vorstehenden galenischen Zubereitungen eingesetzt werden.

0185259

## Patentansprüche

1. Pteridine der allgemeinen Formel

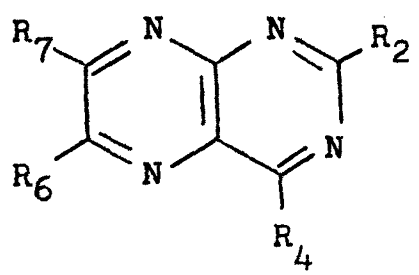

,(I)

in der

$R_2$ die Piperazino- oder N-Formyl-piperazinogruppe,

$R_4$ eine Dialkylamino-, Phenylalkylamino-, N-Alkyl-phenylalkylamino-, Pyrrolidino-, Piperidino-, Morpholino-, Thiomorpholino-, 1-Oxidothiomorpholino-, Thiazolidino- oder 1-Oxidothiazolidinogruppe,

$R_6$ ein Wasserstoffatom, eine Alkyl- oder Phenylgruppe und

$R_7$ eine Alkylamino-, Dialkylamino-, Phenylalkylamino-, N-Alkyl-phenylalkylamino-, Piperidino-, Morpholino-, Thiomorpholino-, 1-Oxidothiomorpholino- oder Piperazinogruppe, wobei in den Resten $R_4$, $R_6$ und $R_7$ jeder Alkylteil 1 bis 3 Kohlenstoffatome enthalten kann und gleichzeitig eine oder beide Alkylgruppen mit 2 oder 3 Kohlenstoffatomen der Reste $R_4$ und $R_7$ in 2- oder 3-Stellung jeweils durch eine Hydroxygruppe substituiert sein können, bedeuten, und deren Säureadditionssalze.

2. Pteridine der allgemeinen Formel I gemäß Anspruch 1, in der

$R_4$, $R_6$ und $R_7$ wie im Anspruch 1 definiert sind und

$R_2$ die Piperazinogruppe darstellt, und deren Säureadditionssalze.

3. Pteridine der allgemeinen Formel I gemäß Anspruch 1, in der

$R_2$ die Piperazinogruppe,

$R_4$ die Dimethylamino-, N-Methyl-2-hydroxyethylamino-, Bis(2-hydroxyethyl)-amino-, Benzylamino-, N-Methyl-benzylamino-, Pyrrolidino-, Piperidino-, Morpholino-, Thiomorpholino-, 1-Oxidothiomorpholino-, Thiazolidino- oder 1-Oxidothiazolidinogruppe,

$R_6$ ein Wasserstoffatom, eine Methyl- oder Phenylgruppe und

$R_7$ eine Dimethylamino-, N-Methyl-2-hydroxyethylamino-, Benzylamino-, N-Methyl-benzylamino-, Piperidino-, Morpholino-, Thiomorpholino-, 1-Oxidothiomorpholino- oder Piperazinogruppe bedeuten, und deren Säureadditionssalze.

4. Pteridine der allgemeinen Formel I gemäß Anspruch 1, in der $R_2$ und $R_6$ wie im Anspruch 3 definiert sind,

$R_4$ eine Pyrrolidino-, Piperidino-, Morpholino-, Thiomorpholino-, 1-Oxidothiomorpholino- oder N-Methyl-2-hydroxyethylaminogruppe und

$R_7$ eine Dimethylamino-, Benzylamino-, N-Methyl-benzylamino-, Morpholino-, Thiomorpholino- oder 1-Oxidothiomorpholinogruppe darstellen, und deren Säureadditionssalze.

5. 7-Benzylamino-6-phenyl-2-piperazino-4-thiomorpholinopteridin und dessen Säureadditionssalze.

6. Physiologisch verträgliche Säureadditionssalze mit anorganischen oder organischen Säuren der Verbindungen gemäß den Ansprüchen 2 bis 5.

7. Arzneimittel, enthaltend eine Verbindung gemäß den Ansprüchen 2 bis 5 oder ein physiologisch verträgliches Säureadditionssalz gemäß Anspruch 6 neben einem oder mehreren inerten Trägerstoffen und/oder Verdünnungsmitteln.

8. Verwendung einer Verbindung gemäß den Ansprüchen 2 bis 5 oder eines physiologisch verträglichen Säureadditionssalzes gemäß Anspruch 6 zur Prophylaxe thrombo-embolischer Erkrankungen, zur Prophylaxe der Arteriosklerose, zur Metastasenprophylaxe und zur Hemmung des Tumorwachstums.

9. Verfahren zur Herstellung eines Arzneimittels, dadurch gekennzeichnet, daß auf nichtchemischem Wege eine Verbindung gemäß den Ansprüchen 2 bis 5 oder ein physiologisch verträgliches Säureadditionssalz gemäß Anspruch 6 in einen oder mehreren inerte Trägerstoffe und/oder Verdünnungmittel eingearbeitet wird.

10. Verfahren zur Herstellung der Verbindungen gemäß den Ansprüchen 1 bis 6, dadurch gekennzeichnet, daß

a) eine Verbindung der allgemeinen Formel

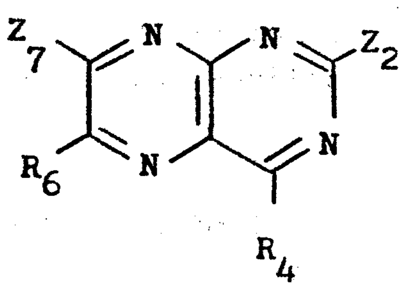

,(II)

in der

$R_4$ und $R_6$ wie in den Ansprüchen 1 bis 5 definiert sind, einer der Reste $Z_2$ oder $Z_7$ eine nukleophil austauschbare Gruppe wie ein Halogenatom darstellt und

der andere der Reste $Z_2$ oder $Z_7$ die für $R_2$ oder $R_7$ in den Ansprüchen 1 bis 5 erwähnten Bedeutungen besitzt oder, falls eine Verbindung der allgemeinen Formel I hergestellt wird, in der die Reste $R_2$ und $R_7$ die gleiche Bedeutung besitzen, auch eine nukleophil austauschbare Gruppe wie ein Halogenatom darstellt, mit einem Amin der allgemeinen Formel

$$H - X \qquad ,(III)$$

in der
X die für $R_2$ oder $R_7$ in den Ansprüchen 1 bis 5 erwähnten Bedeutungen besitzt oder eine durch einen hydrolytisch abspaltbaren Schutzrest geschützte Piperazinogruppe darstellt, umgesetzt und erforderlichenfalls anschließend ein verwendeter Schutzrest abgespalten wird oder

b) zur Herstellung von Verbindungen der allgemeinen Formel I, in der $R_4$ eine 1-Oxidothiomorpholino- oder 1-Oxidothiazolidinogruppe und/oder $R_7$ eine 1-Oxidothiomorpholinogruppe darstellen, eine Verbindung der allgemeinen Formel

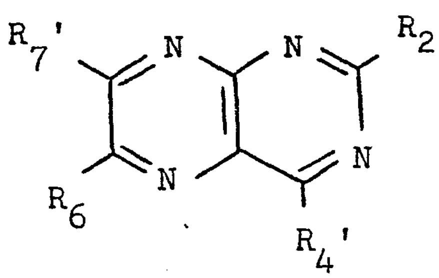

,(IV)

in der
$R_2$ und $R_6$ wie in den Ansprüchen 1 bis 5 definiert sind, $R_4$' und $R_7$' die für $R_4$ bzw. $R_7$ in den Ansprüchen 1

bis 5 erwähnten Bedeutung besitzen, wobei jedoch mindestens einer der Reste $R_4$' oder $R_7$' eine Thiomorpholinogruppe oder $R_4$' auch eine Thiazolidinogruppe darstellen muß, oxidiert wird und

gewünschtenfalls anschließend eine so erhaltene Verbindung der allgemeinen Formel I in ihr Säureadditionssalz insbesondere in ihr physiologisch verträgliches Säureadditionssalz mit einer anorganischen oder organischen Säure übergeführt wird.